# EUROPEAN PATENT SPECIFICATION

(11) **EP 2 948 168 B1**
(45) Date of publication and mention of the grant of the patent: **18.07.2018**
(21) Application number: 13712086.1
(22) Date of filing: 13.03.2013
(51) Int. Cl.: A61K 38/48, A61K 47/51, C07K 14/745, C12N 9/64

(54) **INHIBITION OF TISSUE FACTOR PATHWAY INHIBITOR WITH FACTOR XA DERIVATIVES**
HEMMUNG EINES GEWEBEFAKTORPFAD-INHIBITORS MIT FAKTOR XA-DERIVATEN
INHIBITION D'INHIBITEUR DE LA VOIE DU FACTEUR TISSULAIRE A L'AIDE DE DERIVES DE FACTEUR XA

(30) Priority: 24.01.2013 US 201361756359 P
(43) Date of publication of application: 02.12.2015
(73) Proprietor: Portola Pharmaceuticals, Inc., South San Francisco, CA 94080 (US)
(72) Inventor: LU, Genmin, Burlingame, CA 94010 (US); SINHA, Uma, San Francisco, CA 94127 (US); KARBARZ, Mark, Burlingame, CA 94010 (US); PANDEY, Anjali, Fremont, CA 94555 (US); CONLEY, Pamela, B., Palo Alto, CA 94303 (US)
(74) Representative: J A Kemp
(86) International application number: PCT/US2013/030927
(87) International publication number: WO 2014/116275

(56) References cited:
- WO-A1-2010/117729
- WO-A2-2009/042962

## Description

### FIELD

The present disclosure relates to methods of treating bleeding disorders through the inhibition of the tissue factor pathway inhibitor (TFPI).

### BACKGROUND

Hemostasis relies on the complex coagulation cascade, wherein a series of events mediated by blood clotting factors leads to conversion of prothrombin to thrombin. Factor X (fX) activation is the central event of both the intrinsic and extrinsic pathways of the coagulation cascade. The extrinsic pathway has been proposed as the primary activator of the coagulation cascade. When a blood vessel is damaged, exposed Tissue Factor (TF) interacts with activated Factor VII (fVIIa) to form the "extrinsic complex," which mediates activation of fX. The coagulation cascade is amplified by the intrinsic pathway, during which successive activation of factors XII, XI, IX, and VIII results in formation of the "intrinsic" flXa-fVIIIa complex that also mediates fX activation. Activated fX promotes thrombin formation, which is required for the body to create fibrin and effectively curb bleeding.

Bleeding disorders, such as hemophilia, result from disruption of the blood coagulation cascade. Hemophilia A, the most common type of hemophilia, stems from a deficiency in factor VIII, while hemophilia B is associated with deficiencies in factor IX (fIX). There is currently no cure for hemophilia and other clotting diseases. Factor replacement therapy is the most common treatment for blood coagulation disorders. However, blood clotting factors typically are cleared from the bloodstream shortly after administration. To be effective, a patient must receive frequent intravenous infusions of plasma-derived or recombinant factor concentrates, which is uncomfortable, requires clinical settings, is expensive, and is time consuming. In addition, therapeutic efficacy of factor replacement therapy can diminish drastically upon formation of inhibitory antibodies.

Tissue factor pathway inhibitor (TFPI) is an endogenous protease inhibitor which regulates the extrinsic pathway of blood coagulation. TFPI contains three Kunitz-type protease inhibitor domains, the second Kunitz-domain being a direct inhibitor of fXa. Regulation by a negative feedback mechanism involves an initially formed fXa-TFPI complex, which in turn uses the first Kunitz-domain to bind to fVIIa/TF complex and block further activation of factor X. Therefore, modulating TFPI function can provide an alternative treatment for hemophilia disorders. Indeed, several methods have been published for treating hemophilia by inhibition of TFPI function with RNA aptamer, non-anticoagulant sulfated polysaccharides (NASP), mAbs.
WO 2010/117729 discloses the ability of the modified fXa of SEQ ID NO: 3 to bind and neutralize fXa inhibitors.

### SUMMARY

The present disclosure provides, a method for treating a bleeding disorder in a subject in need thereof, comprising administering to the subject an effective amount of a polypeptide factor Xa (fXa) derivative (a) that comprises a fXa heavy chain that comprises a modification at an active site and (b) that does not include a light chain or comprises a Gla-deficient or des-Gla fXa light chain. The bleeding disorder may be selected from the group consisting of hemophilia A, hemophilia B, a von Willebrand (vWF) disease, a factor XII deficiency and combinations thereof.

Also provided, is a method for improving blood clotting in a subject in need thereof, comprising administering to the subject an effective amount of a polypeptide factor Xa (fXa) derivative (a) that comprises a fXa heavy chain that comprises a modification at an active site and (b) that does not include a light chain or comprises a Gla-deficient or des-Gla fXa light chain.

Another disclosure provides a method for reducing the concentration of circulating tissue factor pathway inhibitor (TFPI) in a subject in need thereof, comprising administering to the subject an effective amount of a polypeptide factor Xa (fXa) derivative (a) that comprises a fXa heavy chain that comprises a modification at an active site and (b) that does not include a light chain or comprises a Gla-deficient or des-Gla fXa light chain.

The subject suffers from a bleeding disorder. In one aspect, the subject is experiencing or at risk of experiencing a bleeding episode.

The modification at the active site may comprise substitution of Ser379 with dehydro-alanine or alanine. The modification may comprise substitution of His236 with alanine and/or substitution of Asp282 with alanine or asparagine.

The fXa heavy chain may further comprise at least one amino acid substitution at amino acid position Arg306, Glu310, Arg347, Lys351, Lys414, or Arg424.

The polypeptide may comprise a peptide linker between the light chain and the heavy chain. In some aspects, the polypeptide is a two-chain polypeptide.

The present invention provides a polypeptide for use in a method for treating hemophilia A or hemophilia B in a subject in need thereof, comprising administering to the subject an effective amount of the polypeptide, wherein the polypeptide comprises SEQ ID NO.3.

In one aspect of the disclosed methods, the methods further comprise administering to the subject an agent selected from the group consisting of BAX499, ARC 19499, mAb2021, NASP and combinations thereof. In one aspect, the method further comprises administering to the subject a recombinant fVIII or fIX. In some aspects, the derivative is conjugated with a moiety capable of extending the circulating half-life of the derivative.

### BRIEF DESCRIPTION OF THE DRAWINGS

Provided as embodiments of this disclosure are drawings which illustrate by exemplification only, and not limitation, wherein:

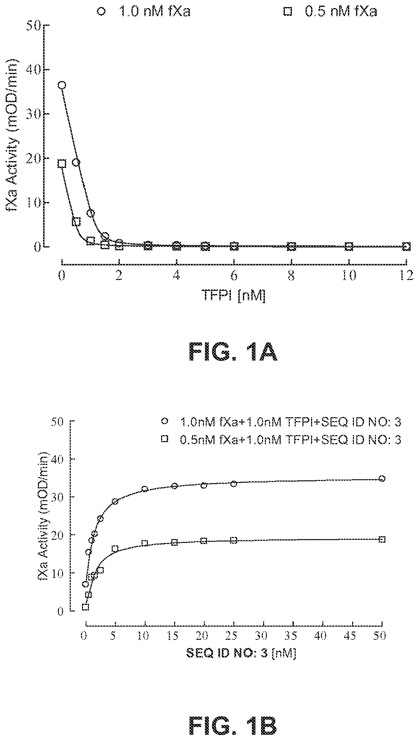

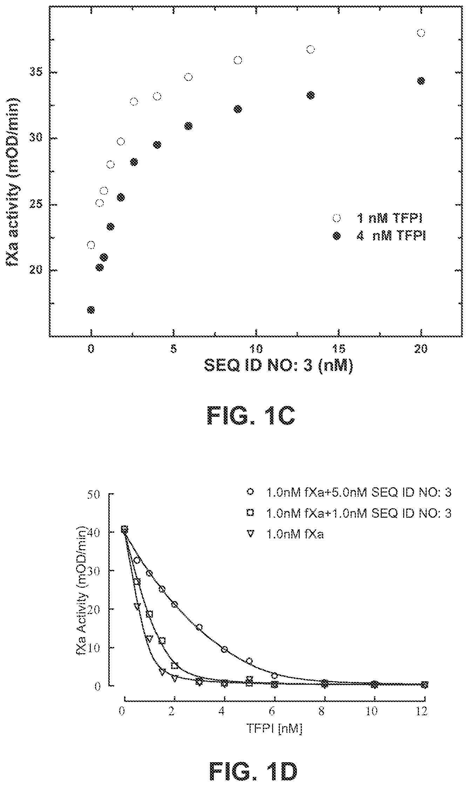

**FIG. 1A-D** show that the fXa derivative (SEQ ID NO: 3) binds to tissue factor pathway inhibitor (TFPI) with sub nano-molar affinity. The presence of TFPI dose-dependently decreased the activity of factor Xa (fXa) (**FIG. 1A**); the inhibition of TFPI of the fXa activity was reversed by increasing the concentration of the fXa derivative for two different concentrations (0.5 and 1 nM) of fXa (**FIG. 1B**) and for two different concentrations (1 and 4 nM) of TFPI (**FIG. 1C**); the inhibition kinetics was studied with different concentrations of TFPI and the fXa derivative (**FIG. 1D**);

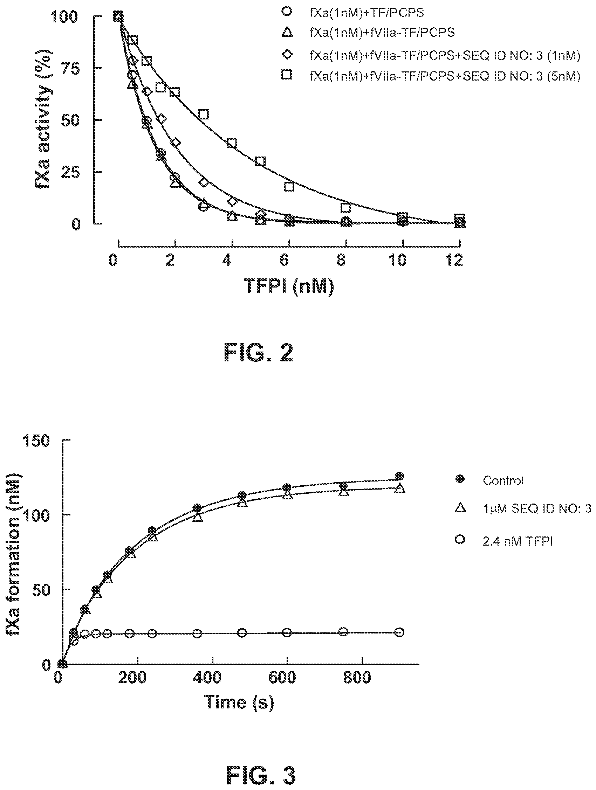

**FIG. 2** shows the effect of the fXa derivative (SEQ ID NO: 3) on TFPI-mediated fXa inhibition in the presence of fVIIa-TF/PCPS;
**FIG. 3** shows the effects of TFPI or the fXa derivative (SEQ ID NO: 3) on fVIIa/TF activity, as measured by fXa formation;

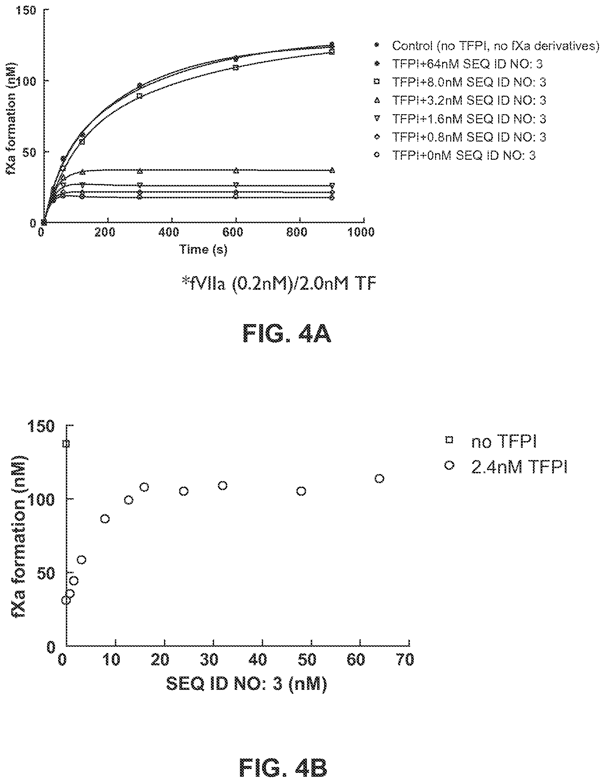

**FIG. 4A-B** show the effect of the fXa derivative (SEQ ID NO: 3) on fX activation by fVIIa/TF in the presence of TFPI. (**A**) Representative progress curves of fXa formation in the presence of increasing concentrations of the fXa derivative. (**B**) Factor Xa formation as a function of the fXa derivative concentrations after incubation for 15 min (the last data points in Panel **A**) in the absence and presence of TFPI;

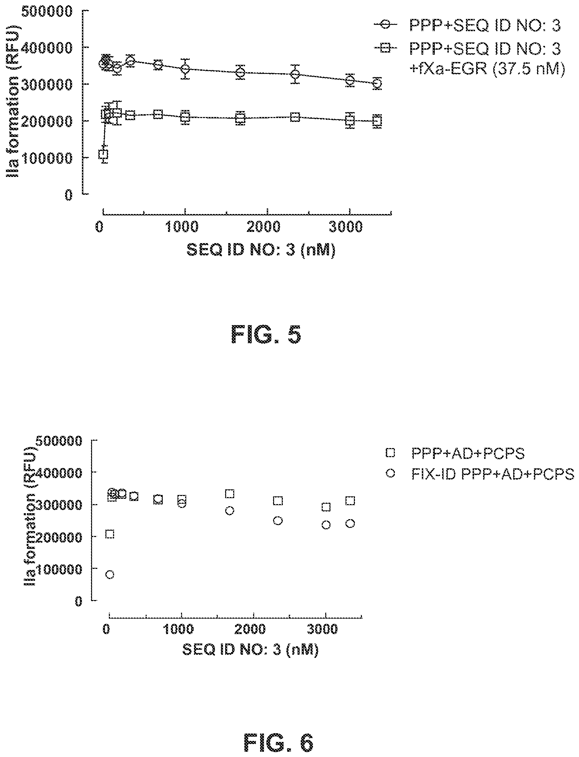

**FIG. 5** shows the effect of the fXa derivative (SEQ ID NO: 3) on thrombin generation in human plasma or human plasma containing 37.5 nM EGR-fXa. The effect of the fXa derivative on thrombin formation could be observed on the background of EGR-fXa, an competitive inhibitor of fXa for the prothrombinase complex; and
**FIG. 6** shows the effect of the fXa derivative (SEQ ID NO: 3) on thrombin generation initiated by low TF (10 pM TF) in normal human plasma or fIX-immuno-depleted human plasma. PCPS was added to maintain the phospholipid concentration due to the lower concentration of TF used in these experiments,vs the standard assay conditions. The effect of the fXa derivative on thrombin formation could be observed with both normal and fIX-deficient plasma.

### DETAILED DESCRIPTION

### I. Definitions

All numerical designations, e.g., pH, temperature, time, concentration, and molecular weight, including ranges, are approximations which are varied (+) or (-) by increments of 0.1. It is to be understood, although not always explicitly stated that all numerical designations are preceded by the term "about". It also is to be understood, although not always explicitly stated, that the reagents described herein are merely exemplary and that equivalents of such are known in the art.

As used in the specification and claims, the singular form "a", "an" and "the" include plural references unless the context clearly dictates otherwise. For example, the term "a pharmaceutically acceptable carrier" includes a plurality of pharmaceutically acceptable carriers, including mixtures thereof.

As used herein, the term "comprising" is intended to mean that the compositions and methods include the recited elements, but do not exclude others. "Consisting essentially of' when used to define compositions and methods, shall mean excluding other elements of any essential significance to the combination for the intended use. Thus, a composition consisting essentially of the elements as defined herein would not exclude trace contaminants from the isolation and purification method and pharmaceutically acceptable carriers, such as phosphate buffered saline, preservatives, and the like. "Consisting of' shall mean excluding more than trace elements of other ingredients and substantial method steps for administering the compositions of this disclosure. Embodiments defined by each of these transition terms are within the scope of this disclosure.

A "bleeding disorder" refers to a disease or condition in a subject with reduced to diminished ability to form blood clots. Blood clotting is required in a condition of tissue injury and thus a reduced clotting condition can be life threatening. Non-limiting examples of bleeding disorders include hemophila A, hemophilia B, a von Willebrand (vWF) disease, a factor XII deficiency and combinations thereof. In some aspects, the subject having a bleeding disorder is not undergoing an anticoagulant therapy. In some aspects, the subject having a bleeding disorder is not receiving administration of a factor Xa inhibitor.

A "composition" is intended to mean a combination of active agent and another compound or composition, inert (for example, a detectable agent or label) or active, such as an adjuvant.

A "pharmaceutical composition" is intended to include the combination of an active agent with a carrier, inert or active, making the composition suitable for diagnostic or therapeutic use *in vitro*, *in vivo* or *ex vivo.*

The term "protein" and "polypeptide" are used interchangeably and in their broadest sense to refer to a compound of two or more subunit amino acids, amino acid analogs or peptidomimetics. The subunits may be linked by peptide bonds. The subunit may be linked by other bonds, e.g., ester, ether, amino, etc. A protein or peptide must contain at least two amino acids and no limitation is placed on the maximum number of amino acids which may comprise a protein's or peptide's sequence. As used herein the term "amino acid" refers to either natural and/or unnatural or synthetic amino acids, including glycine and both the D and L optical isomers, amino acid analogs and peptidomimetics.

It is to be inferred without explicit recitation and unless otherwise intended, that when the present disclosure relates to a polypeptide, protein, polynucleotide or antibody, an equivalent or a biologically equivalent of such is intended within the scope of this disclosure. As used herein, the term "biological equivalent thereof' is intended to be synonymous with "equivalent thereof' which when referring to a reference protein, antibody, polypeptide or nucleic acid, intends those having minimal homology while still maintaining desired structure or functionality. The term "biological equivalent of' a polynucleotide may refer to one that hybridizes under stringent conditions to the reference polynucleotide or its complement. Unless specifically recited herein, it is contemplated that any polynucleotide, polypeptide or protein mentioned herein also includes equivalents thereof. For example, an equivalent intends at least about 80 % homology or identity and alternatively, at least about 85 %, or alternatively at least about 90 %, or alternatively at least about 95 %, or alternatively 98 % percent homology or sequence identity and exhibits substantially equivalent biological activity to the reference protein, polypeptide or nucleic acid.

"Hybridization" refers to hybridization reactions that can be performed under conditions of different "stringency". Conditions that increase the stringency of a hybridization reaction are widely known and published in the art: see, for example, Sambrook, *et al.*, infra. Examples of relevant conditions include (in order of increasing stringency): incubation temperatures of 25° C, 37°C, 50°C, and 68 °C; buffer concentrations of 10 X SSC, 6 X SSC, 1 X SSC, 0.1 X SSC (where SSC is 0.15 M NaCl and 15 mM citrate buffer) and their equivalent using other buffer systems; formamide concentrations of 0%, 25%, 50%, and 75%; incubation times from 5 minutes to 24 hours and washes of increasing duration, increasing frequency, or decreasing buffer concentrations.

A polynucleotide or polynucleotide region (or a polypeptide or polypeptide region) having a certain percentage (for example, 80%, 85%, 90%, or 95%) of "sequence identity" to another sequence means that, when aligned, that percentage of bases (or amino acids) are the same in comparing the two sequences. The alignment and the percent homology or sequence identity can be determined using software programs known in the art, for example those described in Current Protocols in Molecular Biology (Ausubel et al., eds. 1987) Supplement 30, section 7.7.18, Table 7.7.1. Preferably, default parameters are used for alignment. A preferred alignment program is BLAST, using default parameters. In particular, preferred programs are BLASTN and BLASTP, using the following default parameters: Genetic code = standard; filter = none; strand = both; cutoff = 60; expect = 10; Matrix = BLOSUM62; Descriptions = 50 sequences; sort by = HIGH SCORE; Databases = non-redundant, GenBank + EMBL + DDBJ + PDB + GenBank CDS translations + SwissProtein + SPupdate + PIR. Details of these programs can be found at the following Internet address: ncbi.nlm.nih.gov/cgi-bin/BLAST.

"Homology" or "identity" or "similarity" refers to sequence similarity between two peptides or between two nucleic acid molecules. Homology can be determined by comparing a position in each sequence which may be aligned for purposes of comparison. When a position in the compared sequence is occupied by the same base or amino acid, then the molecules are homologous at that position. A degree of homology between sequences is a function of the number of matching or homologous positions shared by the sequences. An "unrelated" or "non-homologous" sequence shares less than 40% identity, or alternatively less than 25% identity, with one of the sequences of the present disclosure.

"Factor Xa" or "fXa" or "fXa protein" is a serine protease in the blood coagulation pathway, which is produced from the inactive factor X (fX). The nucleotide sequence coding human factor X ("fX") can be found in GenBank with accession number "NM_000504." Upon catalytic cleavage of the first 52 residues of the heavy chain, fX is activated to fXa (SEQ ID NO. 1, **Table 1**). FXa contains a light chain and a heavy chain (as shown in **Table 1**). The first 45 amino acid residues (residues 1-45 of SEQ ID NO.1) of the light chain is called the Gla domain because it contains 11 post-translationally modified γ-carboxyglutamic acid residues (Gla). It also contains a short (6 amino acid residues) aromatic stack sequence (residues 40-45 of SEQ ID NO. 1). Chymotrypsin digestion selectively removes the 1-44 residues resulting in Gla-domainless fXa. The serine protease catalytic domain of fXa is located on the C-terminal heavy chain. The heavy chain of fXa is highly homologous to other serine proteases such as thrombin, trypsin, and activated protein C.

"Native fXa" or "wild-type fXa" refers to the fXa naturally present in plasma or being isolated in its original, unmodified form, which possesses the biological activity of activating prothrombin therefore promoting formation of blood clot. The term includes naturally occurring polypeptides isolated from tissue samples as well as recombinantly produced fXa. "Active fXa" refers to fXa having the biological activity of activating prothrombin. "Active fXa" may be a native fXa or modified fXa that retains procoagulant activity.

As used herein, "fXa derivatives" refer to modified fXa proteins that do not compete with fXa in assembling into the prothrombinase complex, have reduced or no procoagulant activities, and yet bind and/or substantially neutralize the tissue factor pathway inhibitor (TFPI). Examples of fXa derivatives are provided in WO2009/042962 and WO/2010/117729, and further provided herein, such as SEQ ID NO: 2, 3, 6 or 7 and biological equivalents thereof.

SEQ ID NO: 2 (**Table 2**) contains 3 mutations relative to the wild type fXa. The first mutation is the deletion of 6-39 aa in the Gla-domain of FX. The second mutation is replacing the activation peptide sequence 143-194 aa with -RKR- (SEQ ID NO: 5). This produces a -RKRRKR- (SEQ ID NO: 4) linker connecting the light chain and the heavy chain. Upon secretion, this linker is cleaved resulting in a two-chain polypeptide, SEQ ID NO: 3 (**Table 3**). The third mutation is mutation of active site residue S379 to an Ala residue. This amino acid substitution corresponds to amino acid 296 and 290 of SEQ ID NOS: 1 and 3, respectively.

The fXa derivatives, with or without the Ser379 modification, may contain modification on the His (to Ala) and/or Asp (to Ala/Asn) residues in the catalytic triad, and a deleted or modified Gla domain (e.g., SEQ ID NOS: 6 and 7, **Tables 4 and 5**). These modifications provide fXa derivatives with reduced enzymatic activity but not competing with fXa in assembling into the prothrombinase complex.

The present disclosure provides a variety of biological equivalents of the disclosed sequences of the fXa derivatives (e.g., SEQ ID NO: 2, 3, 6 and 7), or alternatively polypeptides having certain sequence identity to these fXa derivatives. Such biological equivalents may retain the structural characteristics of these fXa derivatives, that is, a modified active site or heavy chain and a deleted or modified Gla domain. Such biological equivalents may retain the functional features of these fXa derivatives, that is, not competing with fXa in assembling into the prothrombinase complex and having reduced procoagulant activities.

The term "active site" refers to the part of an enzyme or antibody where a chemical reaction occurs. A "modified active site" is an active site that has been modified structurally to provide the active site with increased or deceased chemical reactivity or specificity. Examples of active sites include, but are not limited to, the catalytic domain of human factor X comprising the 235-488 amino acid residues, and the catalytic domain of human factor Xa comprising the 195-448 amino acid residues. Examples of modified active site include, but are not limited to, the catalytic domain of human factor Xa comprising 195-448 amino acid residues in SEQ ID NOS. 2, 3, 6 or 7 with at least one amino acid substitution at position Arg306, Glu310, Arg347, Lys351, Lys414, or Arg424.

"Gla-domainless fXa" or "des-Gla fXa" refers to fXa or a fXa derivative that does not have a Gla-domain and encompasses fXa derivatives bearing other modification(s) in addition to the removal of the Gla-domain. Examples of Gla-domainless fXa include, but are not limited to, fXa derivative lacking all or part of the 1-39 (or 6-39) amino acid residues of SEQ ID NO.1.

"Gla-deficient fXa" refers to fXa or a fXa derivative with reduced number of free side chain γ-carboxyl groups in its Gla-domain. Like Gla-domainless fXa, Gla-deficient fXa can also bear other modifications. Gla-deficient fXa includes uncarboxylated, undercarboxylated and decarboxylated fXa. "Uncarboxylated fXa" or "decarboxylated fXa" refers to fXa derivatives that do not have the γ-carboxy groups of the γ-carboxyglutamic acid residues of the Gla domain, such as fXa having all of its Gla domain γ-carboxyglutamic acid replaced by different amino acids, or fXa having all of its side chain γ-carboxyl removed or masked by means such as amination, esterification, etc. For recombinantly expressed protein, uncarboxylated fXa is, sometimes, also called non-carboxylated fXa. "Undercarboxylated fXa" refers to fXa derivatives having reduced number of γ-carboxy groups in the Gla domain as compared with wild-type fXa, such as fXa having one or more but not all of its Gla domain γ-carboxyglutamic acids replaced by one or more different amino acids, or fXa having at least one but not all of its side chain γ-carboxyl removed or masked by means such as amination and esterification, etc.

**Table 1. Polypeptide sequence of activated human factor X, fXa (SEQ ID NO: 1)**

| Light Chain | | | | | | |
|---|---|---|---|---|---|---|
| 1 | ANSFLEEMKK | GHLERECMEE | TCSYEEAREV | FEDSDKTNEF | WNKYKDGDQC | ETSPCQNQGK |
| 61 | CKDGLGEYTC | TCLEGFEGKN | CELFTRKLCS | LDNGDCDQFC | HEEQNSVVCS | CARGYTLADN |
| 121 | GKACIPTGPY | PCGKQTLER | | | | |

| Heavy Chain | | | | | | |
|---|---|---|---|---|---|---|
| 181 | | IVGGQE | CKDGECPWQA | LLINEENEGF | CGGTILSEFY | ILTAAHCLYQ |
| 241 | AKRFKVRVGD | RNTEQEEGGE | AVHEVEVVIK | HNRFTKETYD | FDIAVLRLKT | PITFRMNVAP |
| 301 | ACLPERDWAE | STLMTQKTGI | VSGFGRTHEK | GRQSTRLKML | EVPYVDRNSC | KLSSSFIITQ |
| 361 | NMFCAGYDTK | QEDACQGDAG | GPHVTRFKDT | YFVTGIVSWG | EGCARKGKYG | IYTKVTAFLK |
| 421 | WIDRSMKTRG | LPKAKSHAPE | VITSSPLK | | | |

**Table 2. Polypeptide sequence of a fXa derivative prior to removal of the -RKRRKR- linker (SEQ ID NO: 2)**

| Light Chain | | | | | | |
|---|---|---|---|---|---|---|
| 1 | ANSFL | | | F | WNKYKDGDQC | ETSPCQNQGK |
| 61 | CKDGLGEYTC | TCLEGFEGKN | CELFTRKLCS | LDNGDCDQFC | HEEQNSVVCS | CARGYTLADN |
| 121 | GKACIPTGPY | PCGKQTLER | | | | |
| Linker | | | | | | |
| | RKRRKR | | | | | |

| Heavy Chain | | | | | | |
|---|---|---|---|---|---|---|
| 181 | | IVGGQE | CKDGECPWQA | LLINEENEGF | CGGTILSEFY | ILTAAHCLYQ |
| 241 | AKRFKVRVGD | RNTEQEEGGE | AVHEVEVVIK | HNRFTKETYD | FDIAVLRLKT | PITFRMNVAP |
| 301 | ACLPERDWAE | STLMTQKTGI | VSGFGRTHEK | GRQSTRLKML | EVPYVDRNSC | KLSSSFIITQ |
| 361 | NMFCAGYDTK | QEDACQGD**A**G | GPHVTRFKDT | YFVTGIVSWG | EGCARKGKYG | IYTKVTAFLK |
| 421 | WIDRSMKTRG | LPKAKSHAPE | VITSSPLK | | | |

**Table 3. Polypeptide sequence of a fXa derivative after removal of the -RKRRKR- linker (SEQ ID NO: 3)**

| Light Chain | | | | | | |
|---|---|---|---|---|---|---|
| 1 | ANSFL | | | F | WNKYKDGDQC | ETSPCQNQGK |
| 61 | CKDGLGEYTC | TCLEGFEGKN | CELFTRKLCS | LDNGDCDQFC | HEEQNSVVCS | CARGYTLADN |
| 121 | GKACIPTGPY | PCGKQTLER | | | | |

| Heavy Chain | | | | | | |
|---|---|---|---|---|---|---|
| 181 | | IVGGQE | CKDGECPWQA | LLINEENEGF | CGGTILSEFY | ILTAAHCLYQ |
| 241 | AKRFKVRVGD | RNTEQEEGGE | AVHEVEVVIK | HNRFTKETYD | FDIAVLRLKT | PITFRMNVAP |
| 301 | ACLPERDWAE | STLMTQKTGI | VSGFGRTHEK | GRQSTRLKML | EVPYVDRNSC | KLSSSFIITQ |
| 361 | NMFCAGYDTK | QEDACQGD**A**G | GPHVTRFKDT | YFVTGIVSWG | EGCARKGKYG | IYTKVTAFLK |
| 421 | WIDRSMKTRG | LPKAKSHAPE | VITSSPLK | | | |

**Table 4. Polypeptide sequence of a fXa derivative prior to removal of the -RKRRKR- linker (SEQ ID NO: 6)**

| Light Chain | | | | | | |
|---|---|---|---|---|---|---|
| 1 | ANSFL | | | F | WNKYKDGDQC | ETSPCQNQGK |
| 61 | CKDGLGEYTC | TCLEGFEGKN | CELFTRKLCS | LDNGDCDQFC | HEEQNSVVCS | CARGYTLADN |
| 121 | GKACIPTGPY | PCGKQTLER | | | | |
| Linker | | | | | | |
| | RKRRKR | | | | | |

| Heavy Chain | | | | | | |
|---|---|---|---|---|---|---|
| 181 | | IVGGQE | CKDGECPWQA | LLINEENEGF | CGGTILSEFY | ILTAA**H**CLYQ |
| 241 | AKRFKVRVGD | RNTEQEEGGE | AVHEVEVVIK | HNRFTKETYD | FDIAVLRLKT | PITFRMNVAP |
| 301 | ACLPERDWAE | STLMTQKTGI | VSGFGRTHEK | GRQSTRLKML | EVPYVDRNSC | KLSSSFIITQ |
| 361 | NMFCAGYDTK | QEDACQGD**S**G | GPHVTRFKDT | YFVTGIVSWG | EGCARKGKYG | IYTKVTAFLK |
| 421 | WIDRSMKTRG | LPKAKSHAPE | VITSSPLK | | | |

**Table 5. Polypeptide sequence of a fXa derivative after removal of the -RKRRKR- linker (SEQ ID NO: 7)**

| Light Chain | | | | | | |
|---|---|---|---|---|---|---|
| 1 | ANSFL | | | F | WNKYKDGDQC | ETSPCQNQGK |
| 61 | CKDGLGEYTC | TCLEGFEGKN | CELFTRKLCS | LDNGDCDQFC | HEEQNSVVCS | CARGYTLADN |
| 121 | GKACIPTGPY | PCGKQTLER | | | | |

| Heavy Chain | | | | | | |
|---|---|---|---|---|---|---|
| 181 | | IVGGQE | CKDGECPWQA | LLINEENEGF | CGGTILSEFY | ILTAA**H**CLYQ |
| 241 | AKRFKVRVGD | RNTEQEEGGE | AVHEVEVVIK | HNRFTKETYD | F**D**IAVLRLKT | PITFRMNVAP |
| 301 | ACLPERDWAE | STLMTQKTGI | VSGFGRTHEK | GRQSTRLKML | EVPYVDRNSC | KLSSSFIITQ |
| 361 | NMFCAGYDTK | QEDACQGD**S**G | GPHVTRFKDT | YFVTGIVSWG | EGCARKGKYG | IYTKVTAFLK |
| 421 | WIDRSMKTRG | LPKAKSHAPE | VITSSPLK | | | |

The term "active site" refers to the part of an enzyme or antibody where a chemical reaction occurs. A "modified active site" is an active site that has been modified structurally to provide the active site with increased or decreased chemical reactivity or specificity. Examples of active sites include, but are not limited to, the catalytic domain of human factor X comprising the 235-488 amino acid residues, and the catalytic domain of human factor Xa comprising the 195-448 amino acid residues. Examples of modified active site include, but are not limited to, the catalytic domain of human factor Xa comprising 195-448 amino acid residues in SEQ ID NO: 1 with at least one amino acid substitution at position Arg306, Glu310, Arg347, Lys351, Lys414, or Arg424.

### II. Compositions

It is discovered that a factor Xa (fXa) derivative, SEQ ID NO: 3, was able to bind the tissue factor pathway inhibitor (TFPI) at high affinity and diminish TFPI's ability to inhibit the extrinsic coagulation pathway. Such a discovery was unexpected as it was known that TFPI binds fXa at a much higher affinity when fXa is present in a prothrombinase complex than when fXa is alone. The fXa derivative, however, is unable to participate in the formation of a prothrombinase complex due to its lack of the Gla domain. It is also unexpected, and surprising, to find that the fXa derivative-TFPI complex is unable to participate in the negative feedback regulation of the fVIIa/TF function, possibly due to its lack of the Gla domain.

As the physiological concentration of the TFPI protein in a human subject is low (about 2.4 nM), high-affinity binding would be required for an agent to effectively inhibit TFPI *in vivo.* Such an unexpected finding, therefore, indicates that the fXa derivative, as well as its structural and functional equivalents, is a suitable agent for treating bleeding disorders in a subject by inhibiting TFPI in the subject.

Further, such an agent presents unique advantages in a clinical setting as compared to other TFPI inhibitors and other fXa derivatives. First, compared to other TFPI inhibitors such as small molecules and antibodies, the fXa derivative is safer because it resembles a native protein, as evidenced in clinical trials. Second, compared to other fXa derivatives, the fXa derivatives disclosed herein avoid potential clinical complications because they lack catalytic activities. Therefore, the fXa derivatives can neutralize TFPI without interfering with other biological events. As demonstrated in the examples, among all major plasma proteins, the fXa derivatives only bind to TFPI.

The present disclosure provides experimental data from several approaches used to assess the binding of the fXa derivative to TFPI and interference with TFPI activity. Two different fXa chromogenic assays measured the effect of TFPI on purified fXa activity in the absence and presence of the fXa derivative. The effect of the fXa derivative on TFPI-induced inhibition of fXa activity in the presence of fVIIa/TF was also measured. Further, in order to measure the more physiologically relevant activity of TFPI on coagulation protein complexes, the effect of fXa derivative on TFPI function was characterized by measuring fX activation by fVIIa/TF.

TFPI inhibition of coagulation is achieved through two mechanisms. First, TFPI can bind fXa and directly inhibit fXa activity. Second, the TFPI-fXa complex can bind to and inhibit the fVIIa/TF complex, thus inhibiting the extrinsic pathway of coagulation. Introduction of the fXa derivative, or its biological equivalents, as the experimental data show, can interfere with these processes and diminishes the TFPI inhibition of the coagulation process. Clinically, the data indicate that the fXa derivative and its biological equivalents can be used to treat a bleeding disorder through such a mechanism.

Accordingly, the present disclosure provides a method of improving blood clotting in a subject in need thereof. Improvement of blood clotting is particularly useful in subjects that are at risk of suffering from a bleeding episode or having a bleeding disorder condition. Also provided, therefore, are methods for treating a bleeding disorder in a subject in need thereof.

The methods entail administering to the subject an amount of a fXa derivative of the present disclosure. In one aspect, the administered fXa derivative is sufficient to neutralize from about 20% to about 95% of circulating TFPI activity (e.g., active TFPI in circulation). Alternatively, in one aspect, the fXa derivative neutralizes less than about 90%, or about 85%, 80%, 75%, 70%, 65%, 60%, 55%, 50%, 45%, 40%, 35%, 30%, or 25% of the circulating TFPI activity. In another aspect, the fXa derivative neutralizes greater than about 25%, or about 30%, 35%, 40%, 45%, 50%, 55%, 60%, 65%, 70%, 75%, 80%, or 85% of the circulating TFPI activity.

In some aspects, the fXa derivative administered reaches a circulating concentration that is at least about 50% of the circulating concentration of the TFPI in the subject. Alternatively, the fXa derivative administered reaches a circulating concentration that is at least about 75%, 100%, 1.5 fold, 2 fold, 3 fold, 4 fold, 5 fold, 6 fold, 7 fold, 8 fold, 9 fold, 10 fold, 15 fold, 20 fold, 30 fold, 40 fold or 50 fold of the circulating concentration of the TFPI in the subject. In one aspect, the fXa derivative administered reaches a circulating concentration that is not higher than 1000 fold, 900 fold, 800 fold, 700 fold, 600 fold, 500 fold, 400 fold, 300 fold, 200 fold, 100 fold, 90 fold, 80 fold, 70 fold, 60 fold, 50 fold, 40 fold, 30 fold, 20 fold, 15 fold, 10 fold, 9 fold, 8 fold, 7 fold, 6 fold, 5 fold, 4 fold, 3 fold, 2 fold or 100% of the circulating concentration of the TFPI in the subject.

In some aspects, the fXa derivative administered is at least about 0.001 mg/Kg body weight, or alternatively at least about 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9 or 1 mg/Kg body weight. In some aspects, the fXa derivative administered is not higher than about 1 mg/Kg body weight, or alternatively not higher than about 0.9, 0.8, 0.7, 0.6, 0.5, 0.4, 0.3, 0.2, 0.1 or 0.05 mg/Kg body weight. In one aspect, the fXa derivative administered is from about 0.001 mg/Kg to about 1 mg/Kg. In another aspect, the fXa derivative administered is from about 0.01 mg/Kg to about 0.1 mg/Kg.

Provided is a method of improving blood clotting in a subject in need thereof. The fXa derivative administered may be at least about 0.001 mg/Kg body weight, or alternatively at least about 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9 or 1 mg/Kg body weight. The fXa derivative administered may be not higher than about 1 mg/Kg body weight, or alternatively not higher than about 0.9, 0.8, 0.7, 0.6, 0.5, 0.4, 0.3, 0.2, 0.1 or 0.05 mg/Kg body weight. The fXa derivative administered may be from about 0.001 mg/Kg to about 1 mg/Kg. The fXa derivative administered may be from about 0.01 mg/Kg to about 0.1 mg/Kg.

The method may entail administering to the subject a therapeutically effective amount of a fXa derivative. In one aspect, the fXa derivative is administered at least about 5 minutes after the bleeding (blood loss) initiated. Alternatively, the fXa derivative is administered at least about 10 minutes, 15 minutes, 20 minutes, 25 minutes or 30 minutes after the bleeding has initiated. In the event such as when a blood loss episode is predictable, the fXa derivative can also be administered prior to the episode. Therefore, in some aspects, the fXa derivative is administered at least about 5 minutes, 10 minutes, 15 minutes, 20 minutes, 25 minutes or 30 minutes before the bleeding has initiated.

In some embodiments, the method further entails administering to the subject an agent suitable for binding or neutralizing TFPI or suitable for treating a bleeding disorder. Non-limiting examples of such agents include BAX499 (Gorczyca et al., J Thromb Haemost. 10(8):1581-90, 2012), ARC19499 (Waters et al., Blood, 117(20):5514-22, 2011), mAb2021 (Hilden et al., Blood, 119(24):5871-8, 2012), NASP (Liu et al., Thromb Haemost. 95:68-76, 2006), and combinations thereof.

In some embodiments, the method further entails administering to the subject a recombinant fVIII or flX. Recombinant fVIII and fIX can be readily prepared with conventional molecular biology methods.

In some embodiments, the fXa derivative is conjugated with a moiety capable of extending the circulating half-life of the derivative.

Compositions that contain a fXa derivative that are useful for the disclosed methods are also disclosed. In some embodiments, the compositions further include a pharmaceutically acceptable carrier.

"Pharmaceutically acceptable carriers" refers to any diluents, excipients, or carriers that may be used in the compositions of the disclosure. Pharmaceutically acceptable carriers include saline, ion exchangers, alumina, aluminum stearate, lecithin, serum proteins, such as human serum albumin, buffer substances, such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat. Suitable pharmaceutical carriers are described in Remington's Pharmaceutical Sciences, Mack Publishing Company, a standard reference text in this field. They are preferably selected with respect to the intended form of administration, that is, oral tablets, capsules, elixirs, syrups and the like, and consistent with conventional pharmaceutical practices.

The formulations of the disclosure can be manufactured by methods well known in the art such as conventional granulating, mixing, dissolving, encapsulating, lyophilizing, or emulsifying processes, among others. Compositions may be produced in various forms, including granules, precipitates, or particulates, powders, including freeze dried, rotary dried or spray dried powders, amorphous powders, injections, emulsions, elixirs, suspensions or solutions. Formulations may optionally contain stabilizers, pH modifiers, surfactants, bioavailability modifiers and combinations of these.

In one embodiment, the fXa derivative is lyophilized. Methods for lyophilizing polypeptides are well known in the art.

Pharmaceutical formulations may also be prepared as liquid suspensions or solutions using a sterile liquid, such as oil, water, alcohol, and combinations thereof. Pharmaceutically suitable surfactants, suspending agents or emulsifying agents, may be added for oral or parenteral administration. Suspensions may include oils, such as peanut oil, sesame oil, cottonseed oil, corn oil and olive oil. Suspension preparation may also contain esters of fatty acids, such as ethyl oleate, isopropyl myristate, fatty acid glycerides and acetylated fatty acid glycerides. Suspension formulations may include alcohols, such as ethanol, isopropyl alcohol, hexadecyl alcohol, glycerol and propylene glycol. Ethers, such as poly(ethyleneglycol), petroleum hydrocarbons, such as mineral oil and petrolatum, and water may also be used in suspension formulations.

The formulations are for administration to a mammal, preferably a human being. Such formulations of the disclosure may be administered in a variety of ways, preferably parenterally.

The term "parenteral" as used herein includes subcutaneous, intravenous, intramuscular, intra-articular, intra-synovial, intrasternal, intrathecal, intrahepatic, intralesional and intracranial injection or infusion techniques. However, in cases where the fXa inhibitor being neutralized has a long plasma half life, a continuous infusion or a sustained release formulation may be required to bind to the fXa inhibitor and such free up the active fXa prior to the clearance of the fXa inhibitor from the body. Therefore, in one aspect, the formulation is administered to the subject as a bolus. In another aspect, the formulation is administered by infusion. In another aspect, the formulation is administered by a combination of bolus and infusion.

Sterile injectable forms of the compositions of this disclosure may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents which are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation. Compositions may be formulated for parenteral administration by injection such as by bolus injection or continuous infusion. A unit dosage form for injection may be in ampoules or in multi-dose containers.

In addition to dosage forms described above, pharmaceutically acceptable excipients and carriers and dosage forms are generally known to those skilled in the art and are included in the disclosure. It should be understood that a specific dosage and treatment regimen for any particular patient will depend upon a variety of factors, including the activity of the specific fXa derivative employed, the age, body weight, general health, sex and diet, renal and hepatic function of the patient, and the time of administration, rate of excretion, drug combination, judgment of the treating physician or veterinarian and severity of the particular disease being treated.

Polypeptides comprising the amino acid sequences of the disclosure can be prepared by expressing polynucleotides encoding the polypeptide sequences of this disclosure in an appropriate host cell. This can be accomplished by methods of recombinant DNA technology known to those skilled in the art. Accordingly, this disclosure also provides methods for recombinantly producing the polypeptides of this disclosure in a eukaryotic or prokaryotic host cells. The proteins and polypeptides of this disclosure also can be obtained by chemical synthesis using a commercially available automated peptide synthesizer such as those manufactured by Perkin Elmer/Applied Biosystems, Inc., Model 430A or 431A, Foster City, CA, USA. The synthesized protein or polypeptide can be precipitated and further purified, for example by high performance liquid chromatography (HPLC). Accordingly, this disclosure also provides a process for chemically synthesizing the proteins of this disclosure by providing the sequence of the protein and reagents, such as amino acids and enzymes and linking together the amino acids in the proper orientation and linear sequence.

It is known to those skilled in the art that modifications can be made to any peptide to provide it with altered properties. Polypeptides of the disclosure can be modified to include unnatural amino acids. Thus, the peptides may comprise D-amino acids, a combination of D- and L-amino acids, and various "designer" amino acids (e.g., β-methyl amino acids, C-α-methyl amino acids, and N-α-methyl amino acids, etc.) to convey special properties to peptides. Additionally, by assigning specific amino acids at specific coupling steps, peptides with α-helices, β turns, β sheets, α-tums, and cyclic peptides can be generated. Generally, it is believed that α-helical secondary structure or random secondary structure is preferred.

Subunits of polypeptides that confer useful chemical and structural properties may be chosen. For example, peptides comprising D-amino acids may be resistant to L-amino acid-specific proteases *in vivo.* Modified compounds with D-amino acids may be synthesized with the amino acids aligned in reverse order to produce the peptides of the disclosure as retro-inverso peptides. In addition, the present disclosure envisions preparing peptides that have better defined structural properties, and the use of peptidomimetics, and peptidomimetic bonds, such as ester bonds, to prepare peptides with novel properties. A peptide may be generated that incorporates a reduced peptide bond, *i.e.*, R₁-CH₂NH-R₂, where R₁, and R₂ are amino acid residues or sequences. A reduced peptide bond may be introduced as a dipeptide subunit. Such a molecule would be resistant to peptide bond hydrolysis, e.g., protease activity. Such molecules would provide ligands with unique function and activity, such as extended half-lives *in vivo* due to resistance to metabolic breakdown, or protease activity. Furthermore, it is well known that in certain systems constrained peptides show enhanced functional activity (Hruby (1982) Life Sciences 31:189-199 and Hruby et al. (1990) Biochem J. 268:249-262); the present disclosure provides a method to produce a constrained peptide that incorporates random sequences at all other positions.

The following non-classical amino acids may be incorporated in the peptides of the disclosure in order to introduce particular conformational motifs:
1,2,3,4-tetrahydroisoquinoline-3-carboxylate (Kazrnierski et al. (1991) J. Am. Chem. Soc. 113:2275-2283); (2S,3S)-methyl-phenylalanine, (2S,3R)- methyl-phenylalanine, (2R,3S)-methyl-phenylalanine and (2R,3R)-methyl-phenylalanine (Kazmierski and Hruby (1991) Tetrahedron Lett. 32(41):5769-5772); 2-aminotetrahydronaphthalene-2- carboxylic acid (Landis (1989) Ph.D. Thesis, University of Arizona); hydroxy-1,2,3,4-tetrahydroisoquinoline-3-carboxylate (Miyake et al. (1989) J. Takeda Res. Labs. 43:53-76) histidine isoquinoline carboxylic acid (Zechel et al. (1991) Int. J. Pep. Protein Res. 38(2):131-138); and HIC (histidine cyclic urea), (Dharanipragada et al. (1993) Int. J. Pep. Protein Res. 42(1):68-77) and (Dharanipragada et al. (1992) Acta. Crystallogr. C. 48:1239-1241).

The following amino acid analogs and peptidomimetics may be incorporated into a peptide to induce or favor specific secondary structures: LL-Acp (LL-3-amino-2-propenidone-6-carboxylic acid), a β-turn inducing dipeptide analog (Kemp et al. (1985) J. Org. Chem. 50:5834-5838); β-sheet inducing analogs (Kemp et al. (1988) Tetrahedron Lett. 29:5081-5082); β-turn inducing analogs (Kemp et al. (1988) Tetrahedron Lett. 29:5057-5060); α-helix inducing analogs (Kemp et al. (1988) Tetrahedron Lett. 29:4935-4938); α-turn inducing analogs (Kemp et al. (1989) J. Org. Chem. 54:109:115); analogs provided by the following references: Nagai and Sato (1985) Tetrahedron Lett. 26:647-650; and DiMaio et al. (1989) J. Chem. Soc. Perkin Trans. p. 1687; a Gly-Ala turn analog (Kahn et al. (1989) Tetrahedron Lett. 30:2317); amide bond isostere (Clones et al. (1988) Tetrahedron Lett. 29:3853-3856); tetrazole (Zabrocki et al. (1988) J. Am. Chem. Soc. 110:5875-5880); DTC (Samanen et al. (1990) Int. J. Protein Pep. Res. 35:501:509); and analogs taught in Olson et al. (1990) J. Am. Chem. Sci. 112:323-333 and Garvey et al. (1990) J. Org. Chem. 56:436. Conformationally restricted mimetics of beta turns and beta bulges, and peptides containing them, are described in U.S. Patent No. 5,440,013, issued August 8, 1995 to Kahn.

It is known to those skilled in the art that modifications can be made to any peptide by substituting one or more amino acids with one or more functionally equivalent amino acids that does not alter the biological function of the peptide. The amino acid may be substituted by an amino acid that possesses similar intrinsic properties including, but not limited to, hydrophobicity, size, or charge. Methods used to determine the appropriate amino acid to be substituted and for which amino acid are known to one of skill in the art. Non-limiting examples include empirical substitution models as described by Dahoff et al. (1978) In Atlas of Protein Sequence and Structure Vol. 5 suppl. 2 (ed. M.O. Dayhoff), pp. 345-352. National Biomedical Research Foundation, Washington DC; PAM matrices including Dayhoff matrices (Dahoff *et al.* (1978), *supra*, or JTT matrices as described by Jones et al. (1992) Comput. Appl. Biosci. 8:275-282 and Gonnet et al. (1992) Science 256:1443-1145; the empirical model described by Adach and Hasegawa (1996) J. Mol. Evol. 42:459-468; the block substitution matrices (BLOSUM) as described by Henikoff and Henikoff (1992) Proc. Natl. Acad. Sci. USA 89:10915-10919; Poisson models as described by Nei (1987) Molecular Evolutionary Genetics. Columbia University Press, New York.; and the Maximum Likelihood (ML) Method as described by Müller et al. (2002) Mol. Biol. Evol. 19:8-13.

### EXAMPLES

The disclosure is further understood by reference to the following examples, which are intended to be purely exemplary of the disclosure.

Unless otherwise stated all temperatures are in degrees Celsius. Also, in these examples and elsewhere, abbreviations have the following meanings:
- hr: = hour
- INR: = international normalized ratio
- IV: = intravenous
- kg: = kilogram
- M: = molar
- mg: = milligram
- mg/kg: = milligram/kilogram
- mg/mL: = milligram/milliliter
- min: = minute
- mL: = milliliter
- PCPS: = phosphatidylcholine:phosphatidylserine membranes
- PPP: = platelet poor plasma
- PRP: = platelet rich plasma
- PT: = prothrombin time
- TF: = tissue factor
- TFPI: = tissue factor pathway inhibitor
- U/mL: = units/milliliter
- µL or uL: = microliter
- µM: = micromolar

### Example 1. fXa derivative (SEQ ID NO: 3) binds mainly to TFPI among major plasma proteins

A few major plasma proteins, including tissue factor pathway inhibitor (TFPI), antithrombin III (ATIII), α-2-macroglobulin, α-1-antitrypsin, factor VII (fVII), factor X (fX), prothrombin, and factor V (fV), were tested for their binding affinity to a wild-type factor Xa (fXa) and two individual preparations (Prep 1 and Prep 2) of the fXa derivative (SEQ ID NO: 3). The binding experiment was conducted with a Biacore® 3000 with the fXa derivative immobilized on a CM-5 sensor chip according to manufacturer's instruction.

As shown in **Table 6,** except for TFPI, the fXa derivative, along with the wild-type fXa, did not show any physiological binding affinity to any other major plasma proteins. Compared to fXa, further, the fXa derivative appeared to have an even higher affinity to TFPI (0.64-0.7 vs. 0.81-14.5).

**Table 6. fXa derivative binds mainly to TFPI among major plasma proteins**

| **Protein** | **fXa (nM)** | **fXa derivative Prep 1 (nM)** | **fXa derivative Prep 2 (nM)** |
|---|---|---|---|
| TFPI | 0.81-14.5 | 0.64 | 0.7 |
| ATIII | 1060-13200 | no binding | not tested |
| α-2-Macroglobulin | no binding | no binding | not tested |
| α-1-Antitrypsin | no binding | no binding | not tested |
| FVII | 2970 | 2780 | not tested |
| FX | no binding | no binding | no binding |
| Prothrombin | no binding | no binding | not tested |
| FV | no binding | no binding | not tested |

### Example 2. fXa derivative (SEQ ID NO: 3) binds to TFPI with sub nano-molar affinity

The affinity of TFPI binding to fXa and the fXa derivative (using Prep 2 in Example 1) was determined by kinetics.

A fXa chromogenic assay was used to determine the fXa activity under different conditions.

As **FIG. 1A** shows, TFPI dose-dependently inhibited the fXa activity. The binding kinetics was determined using two different concentrations of the fXa derivative (0.5 nM and 1.0 nM) and escalating doses of TFPI, up to about 12 nM. fXa chromogenic activity was measured following a 2 hour incubation of the reaction mixture. Residual fXa activity was measured by cleavage of the peptidyl substrate Spectrozyme-fXa (100 µM). For fXa at both 0.5 nM and 1 nM, a 2 nM concentration of TFPI was enough to almost completely inhibit the activity of fXa. Such inhibition was also significant when TFPI's concentration was at 1 nM.

In the presence of the fXa derivative, however, TFPI's inhibitory effect was reversed. A concentration of 5 nM fXa derivative was able to maximize the reversal for a TFPI concentration of 1.0 nM (**FIG. 1B**).

In another chromogenic fXa assay containing fXa (1nM), TFPI (1 or 4 nM) and fXa peptide substrate, S-2765 (D-Arg-Gly-Arg-pNA), fXa activity was shown to be dose dependently inhibited by TFPI (**FIG. 1C**). Increasing concentrations of the fXa derivative were able to reverse the inhibition of fXa by TFPI.

The binding kinetics were determined using three different concentrations of the fXa derivative (0, 1 nM, and 5 nM) and escalating doses of TFPI, up to about 12 nM. fXa chromogenic activity was measured following a 2 hour incubation of fXa (1nM). Residual fXa activity was measured by cleavage of the peptidyl substrate Spectrozyme-fXa (100 µM). As shown in **FIG. 1D****,** the fXa derivative dose dependently reversed the inhibitory effect of TFPI. These results are also summarized in **Table 7** with calculated affinity for fXa-TFPI (Kᵢ) and fXa derivative-TFPI (K_{d}) interaction. The table shows that fXa derivative binds to TFPI with sub nano-molar affinity.

**Table 7. Affinity of TFPI to fXa and fXa derivative (Prep 2) Determined by Kinetics**

| **Reaction Components** | **TFPI-fXa K*ᵢ* (nM)** | **TFPI-fXa Derivative (Prep 2) K_{d} (nM)** | **Ratio (K_{d}/Kᵢ)** |
|---|---|---|---|
| FXa+TFPI (**FIG. 1A**) | 0.021±0.001 | N/A | N/A |
| FXa+TFPI+fXa derivative (**FIG. 1C**) | 0.026±0.005 | 0.070±0.017 | 2.69 |
| FXa+TFPI+fXa derivative (**FIG. 1D**)) | 0.033±0.004 | 0.155±0.021 | 4.97 |

### Example 3. fXa derivative (SEQ ID NO: 3) inhibited TFPI's function in the presence of fVIIa-TF

In this study, the interaction of the fXa derivative (SEQ ID NO: 3) with TFPI was characterized by incubating TFPI in the presence or absence of fVIIa/TF. The assay mixture contained fXa (1nM), fXa derivative (0, 1, or 5 nM), TF/PCPS (2nM TF), fVIIa (0 or 1nM) and increasing concentrations of TFPI (0-12nM). Innovin was used as the source of TF and phospholipids. After a 1 hour incubation period at room temperature, fXa activity was determined by measuring cleavage of the peptidyl substrate, Spectrozyme-fXa (100µM), and expressed as % control activity in the absence of TFPI. As shown in **FIG. 2****,** the presence of fVIIa/TF did not change TFPI inhibition of fXa activity. Furthermore, the effects of the fXa derivative on TFPI inhibition in the presence of fVIIa/TF were comparable to **FIG. ID** in the absence of fVIIa/TF. The fXa derivative dose dependently reversed the inhibitory effect of TFPI.

fXa-TFPI complex is a potent inhibitor of fVIIa/TF activity. This study further assessed the potential of fXa derivative to interfere with this activity by measuring fX activation. The experiments were performed to demonstrate the inhibitory action of TFPI on fVIIa/TF activity for the activation of human fX (shown in **FIG. 3**). The fVIIa/TF enzyme complex (E) was formed by premixing fVIIa (0.2nM) with TF/PCPS (2.0 nM TF in the form of Innovin®). The concentration of the enzyme complex was equal to the limiting concentration of fVIIa (0.2 nM).

Following addition of fX (at the physiological concentration of 170 nM), fX plus the fXa derivative or fX plus TFPI to the mixture, aliquots were withdrawn from the incubation mixture at 0-15 minutes. The extent of fXa formation was determined by measuring the cleavage of the peptidyl substrate, Spectrozyme-Xa by the aliquot constituents. The rate of fXa peptidyl substrate hydrolysis by the fXa was converted to actual fXa concentration by comparing it with known fXa standards.

**FIG. 3** shows the formation of fXa by fVIIa/TF over time and inhibition of this reaction by TFPI. Within a few seconds of formation from fX, fXa combined with TFPI (included at the physiological concentration of 2.4 nM) and rapidly inhibited fVIIa/TF activity; hence the fXa concentration and activity are severely diminished compared to when TFPI is absent. This is consistent with the mechanism of action for the inhibition of fVIIa/TF by TFPI, in that the action of TFPI on fVIIa/TF requires prior formation of a fXa-TFPI complex. The fact that the curves in the absence and presence of the fXa derivative are super-imposable demonstrates that the fXa derivative had no effect on the formation of fXa by the fVIIa/TF complex or on fXa activity itself.

In another experiment, fX, TFPI and the fXa derivative were pre-incubated for 30 minutes prior to initiation of reaction. The time course of fXa formation in the presence of the fXa derivative (0-64 nM) is shown in **FIG. 4A** and the dose-response for the fXa derivative reversal of TFPI inhibition observed at 15 minutes is shown in **FIG. 4B****.** Even 1.6 nM the fXa derivative was sufficient to elicit interference with the TFPI system under these experimental conditions, and the effect increased with the increase of the dose of the fXa derivative.

Further, in an assay in which thrombin generation was measured under conditions with reduced thrombin formation by either addition of EGR-fXa or using low TF, where the effects were observed and could be attributed to interaction between the fXa derivative and TFPI. **FIG. 5** shows the effect of the fXa derivative (SEQ ID NO: 3) on thrombin generation initiated by high TF (100 pM TF) in human plasma or human plasma containing 37.5 nM EGR-fXa. The effect of the fXa derivative on thrombin formation could be observed on the background of EGR-fXa, an competitive inhibitor of fXa for the prothrombinase complex. Likewise, **FIG. 6** shows the effect of the fXa derivative (SEQ ID NO: 3) on thrombin generation initiated by low TF (10 pM TF) in normal human plasma or fIX-immuno-depleted human plasma. As expected, fIX-deficient plasma itself has lower thrombin formation compared to normal plasma. The effect of the fXa derivative on thrombin generation could be observed with both normal and fIX-deficient plasma In fact, the fXa derivative is able to fully correct the thrombin formation of fIX-deficient plasma to the same level as the normal plasma.

## Claims

1. A polypeptide for use in a method for treating hemophilia A or hemophilia B in a subject in need thereof, comprising administering to the subject an effective amount of the polypeptide, wherein the polypeptide comprises SEQ ID NO.3.

2. The polypeptide for use in the method of claim 1, wherein the subject is experiencing or at risk of experiencing a bleeding episode.

3. The polypeptide for use in the method of any preceding claim, further comprising administering to the subject an agent selected from the group consisting of BAX499, ARC 19499, mAb2021, NASP and combinations thereof.

4. The polypeptide for use in the method of any preceding claim, further comprising administering to the subject a recombinant fVIII or fIX.

5. The polypeptide for use in the method of any preceding claim, wherein the polypeptide is conjugated with a moiety capable of extending the circulating half-life of the derivative.

## Patentansprüche

1. Polypeptid zur Verwendung in einem Verfahren zum Behandeln von Hämophilie A oder Hämophilie B in einem Subjekt, das dessen bedarf, umfassend ein Verabreichen einer wirksamen Menge des Polypeptids an das Subjekt, wobei das Polypeptid SEQ ID NO 3 umfasst.

2. Polypeptid zur Verwendung im Verfahren nach Anspruch 1, wobei das Subjekt eine Blutung erfährt oder gefährdet ist, eine zu erfahren.

3. Polypeptid zur Verwendung im Verfahren nach einem der vorstehenden Ansprüche, ferner umfassend ein Verabreichen eines Wirkstoffs, ausgewählt aus der Gruppe bestehend aus BAX499, ARC19499, mAb2021, NASP und Kombinationen davon, an ein Subjekt.

4. Polypeptid zur Verwendung im Verfahren nach einem der vorstehenden Ansprüche, ferner umfassend ein Verabreichen eines rekombinanten fVIII oder fIX an das Subjekt.

5. Polypeptid zur Verwendung im Verfahren nach einem der vorstehenden Ansprüche, wobei das Polypeptid mit einer Komponente konjugiert ist, die fähig ist, die Zirkulations-Halbwertszeit des Derivats zu verlängern.

## Revendications

1. Polypeptide destiné à une utilisation dans une méthode de traitement de l'hémophilie A ou l'hémophilie B chez un sujet en ayant besoin, comprenant l'administration au sujet d'une quantité efficace du polypeptide, le polypeptide comprenant la SEQ ID n° : 3.

2. Polypeptide destiné à une utilisation dans une méthode selon la revendication 1, dans lequel le sujet est confronté ou risque d'être confronté à un épisode hémorragique.

3. Polypeptide destiné à une utilisation dans la méthode selon l'une quelconque des revendications précédentes, comprenant en outre l'administration au sujet d'un agent choisi dans le groupe constitué par BAX499, ARC19499, mAb2021, NASP et des combinaisons de ceux-ci.

4. Polypeptide destiné à une utilisation dans la méthode selon l'une quelconque des revendications précédentes, comprenant en outre l'administration au sujet d'un recombinant fVIII ou fIX.

5. Polypeptide destiné à une utilisation dans la méthode selon l'une quelconque des revendications précédentes, le polypeptide étant conjugué à un groupe fonctionnel capable d'étendre la demi-vie circulante du dérivé.
